# EUROPEAN PATENT APPLICATION

(11) **EP 2 752 219 A1**
(43) Date of publication of application: **09.07.2014**
(21) Application number: 14150249.2
(22) Date of filing: 06.01.2014
(51) Int. Cl.: A61M 25/01, A61M 25/00

(54) **Removable clip for catheter**

(30) Priority: 07.01.2013 US 201313735672
(71) Applicant: Cook Medical Technologies LLC, Bloomington, IN 47404 (US)
(72) Inventor: Dunung, Ronak Balwant, Bloomington, IN Indiana 47403 (US); Biltz, Benjamin T., Spencer, 47460 (US); Threlkeld, Corrie, Vernon Hills, IL Illinois 60061 (US)
(74) Representative: Garratt, Peter Douglas

(57) **Abstract**

A removable clip (10) for connection with a catheter (100, 120) for a remote surgical procedure is provided. The clip includes a body (20) and an arcuate concave surface configured to rest upon a central portion (24) of the body and opposed first (30) and second (40) legs that each extend therefrom. A ramp surface (60) extends from the body portion, the ramp surface with a portion that extends at an acute angle with respect to a longitudinal axis through the concave surface. A portion of the ramp surface is configured to extend within an aperture within a side wall of a catheter when the clip is fixed to the side wall of the catheter.

## Description

### TECHNICAL FIELD

The subject disclosure is related to kits and/or components for performing clinical procedures within a remote portion of a patient, with the use of various components that are accessed within a clinical area with a catheter and a wireguide.

### BRIEF SUMMARY

A first representative embodiment of the disclosure is provided. The embodiment includes a removable clip for connection with a catheter. The clip comprises a body and an arcuate concave surface configured to rest upon a central portion of the body and opposed first and second legs that each extend therefrom, and a ramp surface extending from the body portion. The ramp surface includes a portion that extends at an acute angle with respect to a longitudinal axis through the concave surface.

Another representative embodiment of the disclosure is provided. The embodiment includes a kit for rapid exchange with a wireguide. The kit includes an elongate catheter extending between a distal end portion and a proximal end portion with a lumen extending therethrough. The catheter further comprises an aperture disposed through a side wall of the catheter to provide communication with the lumen with the aperture disposed upon the distal end portion of the catheter. A clip is removably mountable to an outer surface of the side wall of the catheter proximate to the aperture. The clip comprises a body with a concave surface defined by a central portion and a first leg that extends therefrom. A ramp portion is fixed to the body with at least a portion of the ramp surface extending at an acute angle with respect to a longitudinal axis of a void defined by the concave surface clip that extends through the concave surface. A portion of the ramp surface is configured to extend through the aperture when the clip is engaged with the side wall of the catheter.

Yet another representative embodiment of the disclosure is provided. The embodiment includes a method for using a wire guide with a catheter. The method includes the steps of providing an elongate catheter extending between a distal end portion and a proximal end portion with a lumen therethrough. An aperture is disposed through a side wall of the catheter. The method further includes the step of connecting a clip to an outer surface of the catheter, wherein the clip comprises a body with a concave surface defined by a central portion of the body and a first leg that extends therefrom, and a ramp portion fixed to the body, with at least a portion of the ramp portion extending at an acute angle with respect to a longitudinal axis of the clip that extends through a void defined by the concave surface, wherein a portion of the ramp surface extends through the aperture. The method further includes the step of threading a wire guide through the lumen within the distal end portion until a tip of the wireguide engages the portion of the ramp surface extending through the aperture and into the lumen and continuing to thread the wireguide through the lumen until the tip of the wire guide extends along the ramp surface through the aperture and out of the catheter.

Advantages of the present disclosure will become more apparent to those skilled in the art from the following description of the preferred embodiments of the disclosure that have been shown and described by way of illustration. As will be realized, the disclosed subject matter is capable of other and different embodiments, and its details are capable of modification in various respects. Accordingly, the drawings and description are to be regarded as illustrative in nature and not as restrictive.

### BRIEF DESCRIPTION OF THE DRAWINGS

FIG. 1 is a perspective view of a removable clip for threading a wireguide attached to a catheter and sheath, with the ramp surface extending within an aperture within the catheter.
FIG. 2 is a perspective view of the clip of FIG. 1.

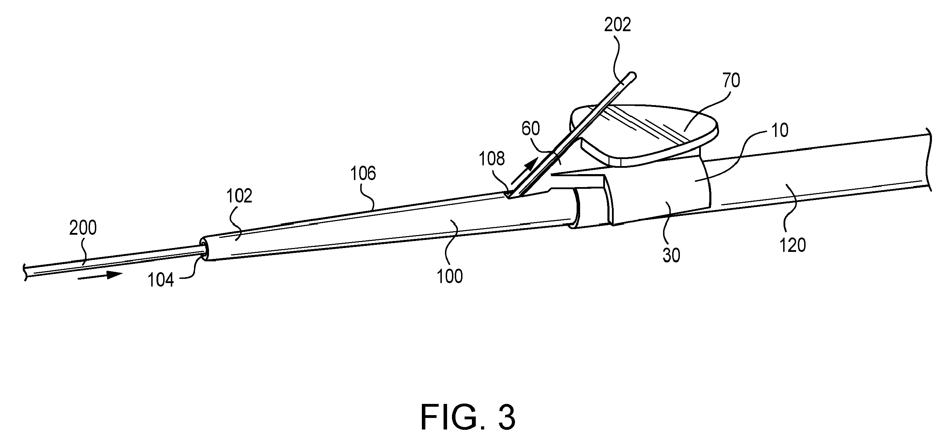

FIG. 3 is a perspective view of a wireguide riding along the ramp surface of the clip and out of the lumen of the catheter.
FIG. 4 is a cross-sectional view of FIG. 3 showing a portion of the wireguide extending from the lumen of the catheter.

### DETAILED DESCRIPTION OF THE DRAWINGS AND THE PRESENTLY PREFERRED EMBODIMENTS

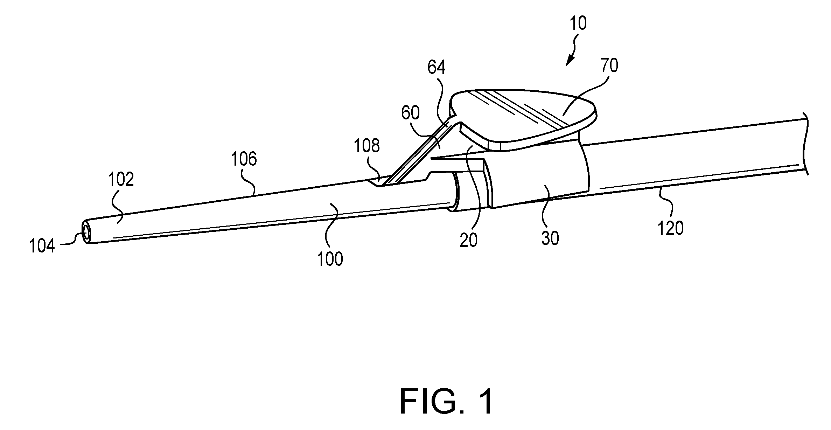

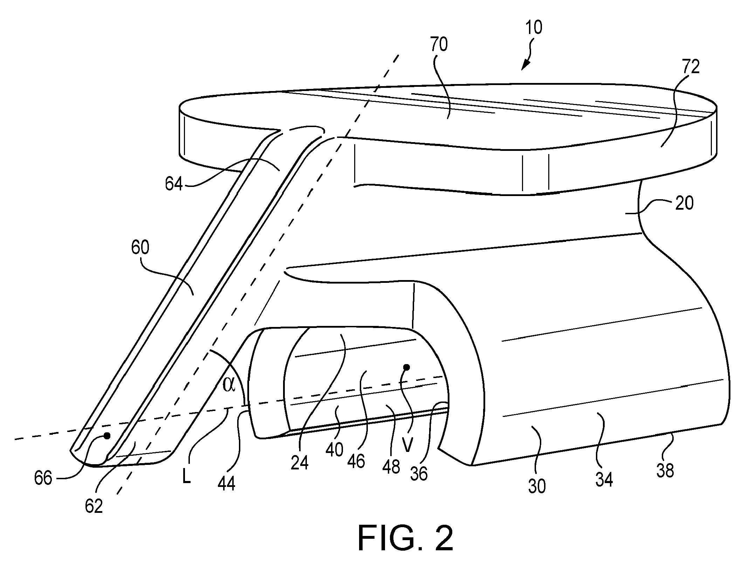

Turning now to FIGs. 1-2, a removable clip 10 is provided. The clip 10 is configured to be removably received upon a catheter 100 (or a sheath 120) that is used for procedures where wire guides 200 are used to steer or direct medical devices into an appropriate surgical location within a patient, and specifically for "rapid exchange" procedures where wire guides 200 are preferably connected and separated from a guiding catheter 100 or sheath 120 while the wire guide 200 and a distal portion 102 of the catheter 100 are both within or proximal to the area of clinical treatment within the patient (and without necessitating the removal of the catheter 100 and wireguide 200 to allow for coupling or decoupling these components). In some embodiments, the clip 10 may be used with a combination catheter 100 and sheath 120 that is slidably disposed thereon.

The clip 10 may include a body 20, a single leg, or in other embodiments first and second legs 30, 40 that extend therefrom, and a ramp surface 60. In some embodiments, the clip 10 may be formed from a single monolithic component, such as a molded or machined component. In other embodiments, the clip 10 may be made from multiple parts that are fixed together. The clip 10 may be made from plastic or metal, but plastic is preferred for reasons of cost and weight.

The opposite first and second legs 30, 40 of the clip each extend from the body 20 of the clip 10, such that the first and second legs 30, 40 may each extend from opposite sides of the body 20. The legs 30, 40 each include an outer surface 34, 44 and an inner surface 36, 46. In some embodiments, the inner surface 36, 46 of each leg 30, 40 includes an arcuate profile, and in some embodiments, the inner surface 36, 46 (and a central portion 24 of the inner surface below the body 20) of each of the first and second legs 30, 40 defines a curve, which may be a continuous or a discontinuous curve. In some embodiments, the curve along the respective inner portions 36, 46 and the central portion 24 defines a continuous curve that forms a constant radius with a respect to a longitudinal axis L that extends through a void V established by the curve. In embodiments with a single leg, the inner surface of the leg may form a continuous or discontinuous curve with the central portion 24, and may be constructed similarly to one of the legs 30, 40 described with respect to embodiments with two legs 30, 40.

In some embodiments, the first and second legs 30, 40 may be connected together, such as with a monolithic ring-like structure, or with a removable connection between the extended ends of the first and second legs 30, 40. In embodiments where the first and second legs 30, 40 of the clip are connected together, the clip is threaded onto the outer surface of the catheter 100 and/or the sheath 120 by sliding the clip over the distal tip of the catheter 100 and advancing the clip proximally until the ramp surface 60 (discussed below) is aligned in registry with the aperture 108 in the side wall of the catheter 100.

In some embodiments, the first and second legs 30, 40 may, in combination extend for an arc length that is less than 360 degrees, but greater than 180 degrees, such as in some embodiments 225 degrees. In other embodiments, the combined first and second legs 30, 40 extend about an arc length from about 190 degrees to about 330 degrees, inclusive of all arc lengths within this range.

In some embodiments, the combined inner surface of the clip 10 may be formed with a curve following a radius that is the same or slightly smaller than or larger than the radius of the catheter 100 or sheath 120. Alternatively, in embodiments where the catheter 100 or sheath 120 receiving the clip 10 does not have a constant diameter, the combined inner surface of the clip may be formed with a profile and size that is the same (or slightly larger than or smaller than) as the outer profile of the catheter 100 or sheath 120.

In other embodiments, the central portion 24 of the inner surface may be formed with a curve (of a radius or profile) that is the same or just larger or smaller than the outer surface of the catheter 100 or sheath 120 (proximate to the aperture 108 upon the catheter 100, as discussed below), while the end portions 38, 48 of one or both of the first and second arms 30, 40 may be formed such that the curve of the inner surface at the respective end portion 38, 48 is biased into a curve (or profile) that is smaller than the outer profile of the catheter 100 or sheath 120, but the one or both of the first and second arms 30, 40 are sufficiently flexible to allow the first and/or second arms to extend over the catheter 100 or sheath 120 to connect the clip 10 to the catheter or sheath 120, proximate to the aperture 108 in the sheath. One of skill in the art upon review of the subject disclosure and figures would understand that the construction of one or both of the first and second arms 30, 40 to a biased position smaller than the outer profile of the catheter 100 or sheath 120, while being sufficiently flexible to deform to allow attachment to the catheter 100 or sheath 120 allows for a reliable, yet removable connection between the catheter 100 (or sheath 120) and the clip 10.

In other embodiments, the clip 10 may be configured to fit directly upon the outer surface of the catheter 100, and therefore the inner surface 36, 46 of one or both of the first and second legs 30, 40 may be at a constant radius along their length (such as the same or slightly larger than the radius of the outer surface of the catheter 100), or the one or both first and second legs 30, 40 may have an inner surface 36, 46 with the same profile as the outer profile catheter 100 (or sheath 120) that receives the clip 10.

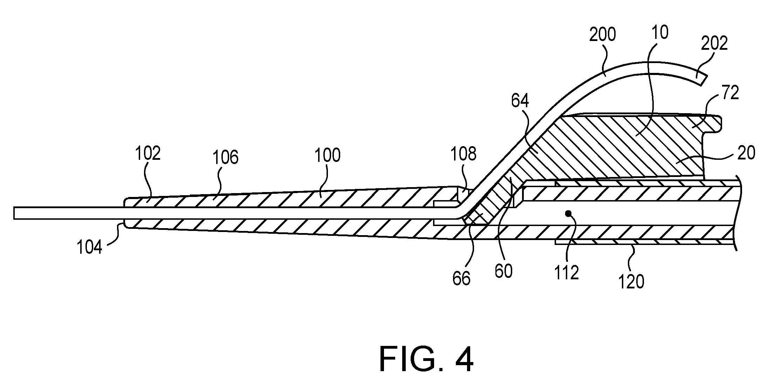

The clip 10 may include a ramp surface 60 that extends at an acute angle α with respect to the longitudinal axis L, which extends through the void V defined by the inner surface of one or both of the first and second legs 30, 40 and the central portion 24. The ramp surface 60 may include an upper portion 64 that is proximate to the body 20 (and may be connected to or monolithically formed with the body 20) and an extended portion 66 that is disposed below the body 20, or below the central portion 24 of the body 20. The extended portion 66 is configured to extend within an aperture 108 defined through a side wall of the catheter 100 such that the extended portion 66 is disposed within the lumen 112 of the catheter (as best shown in FIG. 4).

The ramp surface 60 may extend along a constant angle that is disposed at the acute angle α along its entire length, while in other embodiments, the ramp surface 60 may have a curved profile along its length (or a portion of its length),such as a continuous or a discontinuous curve. For example, in some embodiments, the extended portion 66 of the ramp surface 60 may be defined with a smaller acute angle α with respect to the longitudinal axis L, than the upper portion. In these embodiments, a tip 202 of a wire guide 200 may approach the extended portion 66 of the ramp surface 60 from within the lumen 112 of the catheter 100 and engage and travel easily along the ramp surface 60 due to the small acute angle α, which provides for a smooth transition in direction for the tip 202 of the wire guide 200. As the wire guide continues to travel along the ramp surface 66 (with the acute angle α of the ramp surface 60 increasing), the wire guide 200 is urged from the lumen 112 and out of the catheter 100 through aperture 108 through the side wall of the catheter 100. In embodiments were the angle α is a constant angle, the angle α may be about 45 degrees, or in other embodiments between about 30 degrees to about 60 degrees, inclusive of angles therewithin. As will be appreciated upon a thorough review of this disclosure by one of ordinary skill in the art, the angle α may be a function of the size of the aperture 108 of the catheter, and the geometry of the ramp. It will be appreciated that the aperture 108 when receiving the ramp 60 must be large enough to allow the typical wire guide 200 being used to readily extend therethrough, such as, by way of example, that the geometry and orientation of the ramp surface 60 be design such that a 0.040 inch wide space within the aperture 108 is exposed to allow a typical wire guide 200 to extend therethrough. α

The clip 10 may include an operator 70 that extends from the body 20 and provides a surface or structure to allow the clip 10 to be manipulated to connect or remove the clip 10 from the catheter 100 or sheath 120. In some embodiments, the operator 70 may include one or more wings 72 that extend from the body 20 in a cantilevered manner.

In use, the catheter 100 and clip 10 (and sheath 120, wherein provided) may be used in combination (and sold in a kit together) to allow for convenient threading of a wire guide 200 through a lumen 112 of a catheter from the distal tip (or distal end portion 102, if not through the tip) of the catheter 100. The clip 10 is disposed upon the outer surface of the catheter 100 (or sheath 120), proximate to the aperture 108 through the sidewall of the catheter 100, such that the extended portion 66 of the ramp surface 60 extends through the aperture 108 and into the lumen 112. A wire guide 200 is then threaded into and through the lumen within the distal end portion 102 (and in some embodiments through the tip of the catheter 100 at the distal end) in a direction toward the proximal end of the catheter. The tip 202 of the wire guide 200 contacts the extended portion 66 and travels therealong, such that the direction of travel of the tip 202 of the wire guide is along the direction of the ramp surface 60. As best shown in FIG. 4, the wire guide 200 travels along the ramp surface 60 until the tip 202 and then further portions of the wire guide 200 extend through the aperture 108 and out of the catheter 100. The clip 10 may then be removed from the catheter 100 (or sheath 120) by manipulating the operator 70, and potentially the wing(s) 72 of the operator 70. As the operator 70 is manipulated to pull the clip 10 away from the catheter 100 (or sheath 120), the first and/or second legs 30, 40 of the clip 10 may be urged away from each other to release the connection between the inner surface 36, 46 of the first and second legs 30, 40 from the outer surface of the catheter 100 (sheath 120) to remove the clip 10 therefrom. Once the clip 10 is removed, the catheter 100 (and sheath 120) with the wireguide traveling through a distal portion of the lumen 112 may be inserted into the patient and proximate to the area of clinical interest for the performance of the desired portion of the procedure. During the procedure, the wire guide 200 may be removed from the catheter 100 without removing the catheter 100 from the area of clinical interest (or entirely from the patient) which allows for "rapid exchange" of devices as aided by the wire guide 200.

While the preferred embodiments of the disclosure have been described, it should be understood that the disclosure is not so limited and modifications may be made without departing from the disclosure. The scope of the invention is defined by the appended claims, and all devices that come within the meaning of the claims, either literally or by equivalence, are intended to be embraced therein.

## Claims

1. A removable clip for connection with a catheter, comprising:
a body and an arcuate concave surface configured to rest upon a central portion of the body and opposed first and second legs that each extend therefrom, and
a ramp surface extending from the body portion, the ramp surface with a portion that extends at an acute angle with respect to a longitudinal axis through the concave surface.

2. The clip of claim 1, wherein a portion of the ramp surface extends below the central portion.

3. The clip either of claims 1 or 2, wherein the first and second legs are flexible and are biased to an inner diameter between the first and second legs that is just smaller than an outer diameter of the catheter upon which the clip is configured to be connected.

4. The clip of claim 3, wherein the first and second legs and the central portion define a continuous curve along the concave surface.

5. The clip of any one of the preceding claims, further comprising an operator disposed proximate to the central portion, wherein the operator is configured to be ergonomically manipulated by a user to connect the clip to the catheter and remove the clip from the catheter, preferably wherein the operator comprises a wing that extends from the central portion in a cantilevered manner in parallel to the longitudinal axis.

6. The clip any one of the preceding claims, wherein a portion of the ramp surface is configured to extend within an aperture disposed upon a side wall of a catheter when the clip engages the side wall of the catheter.

7. The clip of any one of the preceding claims, wherein the ramp surface extends at a constant acute angle along its entire length.

8. The clip of claim 2, wherein the ramp surface extends at a changing acute angle along its length, wherein the acute angle is smaller along the portions of the ramp surface that extend below the central portion and larger along the portions of the ramp surface that are at or above the central portion.

9. The clip of claim 8, wherein at least a portion of the ramp surface extends along a continuous curve.

10. The clip of any one of the preceding claims, wherein the concave surface extends cylindrically with respect to the longitudinal axis.

11. The clip of any one of the preceding claims, wherein a portion of the ramp surface extends to a side of the longitudinal axis opposing the central portion.

12. A kit comprising:
an elongate catheter extending between a distal end portion and a proximal end portion with a lumen extending therethrough,
the catheter further comprising an aperture disposed through a side wall of the catheter to provide communication with the lumen,
the aperture disposed upon the distal end portion of the catheter,
the clip of any one of the preceding claims configured to be removably mountable to an outer surface of the side wall of the catheter proximate to the aperture,
wherein at least a portion of the ramp surface extending from the body portion of the clip extends at an acute angle with respect to a longitudinal axis of a void defined by the concave surface clip that extends through the concave surface, and
a portion of the ramp surface configured to extend through the aperture when the clip is engaged with the side wall of the catheter.

13. The kit of claim 12, wherein the combined inner surface of the first leg, the main body, and the second leg of the clip forms a continuous curve therealong.

14. The kit of Claim 12 or Claim 13, wherein the clip is a snap fit to the catheter.

15. A method for assembling a wire guide within a catheter, comprising:
providing an elongate catheter extending between a distal end portion and a proximal end portion with a lumen therethrough, further comprising an aperture disposed through a side wall of the catheter,
connecting a clip to an outer surface of the catheter, wherein the clip comprises a body with a concave surface defined by a central portion of the body and a first leg that extends therefrom, and a ramp portion fixed to the body, with at least a portion of the ramp portion extending at an acute angle with respect to a longitudinal axis of the clip that extends through a void defined by the concave surface, wherein a portion of the ramp surface extends through the aperture,
threading a wire guide through the lumen within the distal end portion until a tip of the wireguide engages the portion of the ramp surface extending through the aperture and into the lumen, continuing to thread the wireguide through the lumen until the tip of the wire guide extends along the ramp surface through the aperture and out of the catheter.
